(19) **Europäisches Patentamt / European Patent Office / Office européen des brevets**

(11) **EP 3 538 502 B1**

(12) **FASCICULE DE BREVET EUROPEEN**

(45) Date de publication et mention de la délivrance du brevet:
**29.10.2025 Bulletin 2025/44**

(21) Numéro de dépôt: **17800751.4**

(22) Date de dépôt: **06.11.2017**

(51) Classification Internationale des Brevets (IPC):
**C07C 1/24** (2006.01)    **C07C 5/27** (2006.01)
**B01J 29/65** (2006.01)    **C07C 11/08** (2006.01)
**C07C 11/09** (2006.01)

(52) Classification Coopérative des Brevets (CPC):
(C-Sets disponibles)
**C07C 1/24; B01J 29/65; C07C 5/2775;**
C07C 2521/08; C07C 2529/65     (Cont.)

(86) Numéro de dépôt international:
**PCT/EP2017/078307**

(87) Numéro de publication internationale:
**WO 2018/087033 (17.05.2018 Gazette 2018/20)**

(54) **PROCEDE DE DESHYDRATATION ISOMERISANTE D'UNE CHARGE ALCOOL PRIMAIRE NON LINEAIRE EN PRESENCE D'INJECTION D'EAU ET D'UN CATALYSEUR COMPRENANT UNE ZEOLITHE DE TYPE FER OU MFS**

VERFAHREN ZUR ISOMERISIERUNGSDEHYDRIERUNG EINES NICHTLINEAREN PRIMÄRALKOHOLAUSGANGSSTOFFES IN GEGENWART VON WASSERINJEKTION UND KATALYSATOR MIT EINEM FER- ODER MFS-ZEOLITH

METHOD FOR THE ISOMERISATION DEHYDRATION OF A NON-LINEAR PRIMARY ALCOHOL FEEDSTOCK IN THE PRESENCE OF WATER INJECTION AND A CATALYST COMPRISING AN FER OR MFS ZEOLITE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.11.2016 FR 1660803**

(43) Date de publication de la demande:
**18.09.2019 Bulletin 2019/38**

(73) Titulaires:
- **IFP Energies nouvelles**
  **92852 Rueil-Malmaison (FR)**
- **Total Research & Technology Feluy**
  **7181 Seneffe (BE)**

(72) Inventeurs:
- **MAURY, Sylvie**
  **69440 Saint Maurice D'Argoire (FR)**
- **COUPARD, Vincent**
  **69100 Villeurbanne (FR)**

- **HEINZ, Thibault**
  **69360 Solaize (FR)**
- **DUPLAN, Guillaume**
  **30340 Saint Julien les Rosiers (FR)**
- **LOPEZ, Joseph**
  **30340 Saint Julien les Rosiers (FR)**
- **NESTERENKO, Nikolai**
  **14000 Nivelles (BE)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**WO-A1-2011/113834     WO-A1-2016/046242**

- **JOSHUA D TAYLOR ET AL: "Dehydration of Fermented Isobutanol for the Production of Renewable Chemicals and Fuels", TOPICS IN CATALYSIS, KLUWER ACADEMIC PUBLISHERS-PLENUM PUBLISHERS, NE, vol. 53, no. 15-18, 22 May 2010 (2010-05-22), pages 1224 - 1230, XP019831608, ISSN: 1572-9028**

Remarques:
Le dossier contient des informations techniques présentées postérieurement au dépôt de la demande et ne figurant pas dans le présent fascicule.

(52) Classification Coopérative des Brevets (CPC): (Cont.)

C-Sets
**C07C 1/24, C07C 11/09;
C07C 5/2775, C07C 11/08**

## Description

## DOMAINE TECHNIQUE DE L'INVENTION

La présente invention concerne un procédé amélioré de production d'alcènes à partir d'une charge comprenant un monoalcool primaire seul ou en mélange, de formule $R-CH_2-OH$, dans lequel R est un radical alkyl non linéaire de formule générale $C_nH_{2n+1}$ où n est un entier compris entre 3 et 20 (tel que l'isobutanol), le procédé opérant en présence d'une quantité d'eau optimale injectée avec la charge. Les performances du procédé sont élevées, notamment en termes de stabilité de la conversion et/ou de la sélectivité en produits recherchés . Cette charge peut être obtenue par des procédés chimiques ou par des procédés fermentaires. Ce procédé met en œuvre un catalyseur mis en forme à base d'une zéolithe comprenant au moins une série de canaux dont l'ouverture est définie par un anneau à 8 atomes d'oxygène (8MR).

Les alcènes obtenus présentent un intérêt important dans le domaine de l'industrie pétrochimique et de la synthèse organique. C'est le cas notamment de l'isobutène, du butène-1 et du butène-2.

## ART ANTÉRIEUR

L'isobutène est une molécule clé en pétrochimie et pour la synthèse d'additifs essence tels que l'ETBE et le MTBE. La grande majorité des publications porte sur la production d'isobutène à partir de butanols linéaires, ceux-ci étant plus facilement produits par voies fermentaires classiques (ABE) que l'isobutanol. De récents développements ont cependant permis d'améliorer fortement les rendements fermentaires en isobutanol, rendant cette charge accessible et disponible à coût attractif.

Le document WO2009/074798 décrit un procédé de conversion du n-butanol en di-isobutène (2,4,4-trimethylpentene), procédé incluant une étape de déshydratation avec isomérisation du n-butanol. Lors de cette étape, il est obtenu au moins 20%pds d'isobutène dans l'effluent et l'exemple montre que environ 31% d'isobutène sont produits.

Le procédé préconise l'ajout d'eau à la charge dans le ratio volumique eau : n-butanol de 1 à 70, ou encore 5-50 ou 10-35, ce qui aurait pour effet d'améliorer la productivité et la sélectivité en isobutène.

Or l'exemple comparatif avec une zéolite ZSM-23 montre que l'ajout d'eau n'apporte pas d'amélioration sur la sélectivité en isobutène par rapport aux butènes totaux. Cette présence d'eau a par contre l'avantage de ne pas faire baisser la sélectivité, et permet donc de ne pas sécher la charge, ce qui est économiquement favorable comme l'indique le brevet.

L'autre zéolite testée est la Theta-1 qui montre une sélectivité en n-butènes de 53% par rapport aux butènes totaux.

Le catalyseur comprend une zéolithe unidirectionnelle sans autres canaux interconnectés. Les zéolithes exemplifiées sont la Theta-1 (de type TON à ouverture de canaux 10MR) et ZSM-23 (de type MTT à ouverture de canaux 10MR). La zéolithe SAPO-11 (également à 10MR) est citée. La ferriérite est également citée sans être exemplifiée, mais d'une part, elle ne correspond pas à l'enseignement de ce document puisqu'elle possède 2 séries de canaux interconnectés (8MR et 10MR) et d'autre part, aucun moyen d'adapter le taux d'isomérie n'est décrit.

Le document WO 2011/113834 décrit la déshydratation et l'isomérisation squelettale simultanée de l'isobutanol en présence de catalyseurs silicates cristallins comprenant au moins des canaux 10MR, désaluminés ou non, modifiés au phosphore ou non, du groupe FER(canaux 8 et 10MR), MWW(10 et 10MR), EUO(10MR), MFS(8 et 10MR), ZSM-48(10MR), MTT(10MR), MFI(10 et 10MR), MEL(10MR) ou TON(10MR) ayant un ratio Si/Al supérieur à 10, tamis moléculaires silicoaluminophosphates du groupe AEL(10MR), ou silice-, zircone-, titane- ou fluor-alumine sur catalyseurs zéolitiques.

Le procédé opère avec une WHSV par rapport à l'alcool d'au moins 1 $h^{-1}$ et une température de 200 à 600°C. L'exemple est réalisé en faisant pa sser une charge isobutanol / eau (ratio pondéral 95 : 5) sur une zéolite FER en poudre de Si/Al 33 à 375°C, 2 bars et à forte WHSV (12.6 $h^{-1}$). Dans ces conditions, la proportion maximale atteinte en n-butènes dans les butènes (isobutène plus butènes linéaires) est de 58.4%, valeur supérieure à celle attendue lors de l'isomérisation de l'isobutène à l'équilibre thermodynamique des butènes.

le document WO2016/046242A1 divulgue un procédé de déshydratation isomérisante d'une charge comprenant de l'isobutanol et 5% d'eau , ledit procédé opérant- en phase gaz à une température moyenne pondérée de 300°C, à une pression de 0,2 MPa et à une PPH (poids par poids par heure) égale a 5 h-1- en présence d'un catalyseur comprenant au moins un liant silicique et une ferriérite.

La déshydratation d'alcools en $C_4$ sur solides acides s'accompagne généralement de l'isomérisation de position de l'alcène formé. Ces deux réactions sont en effet concomitantes, puisque l'isomérisation de position de la double liaison de l'alcène est aussi rapide que la réaction de déshydratation du monoalcool en $C_4$. Dans le cas de l'isobutanol, l'isobutène formé se protonne facilement (formation d'un carbocation tertiaire) et peut ensuite subir des réactions secondaires conduisant à une dégradation de la sélectivité en produit désiré mais également à une désactivation du catalyseur par cokage.

Chadwick et al (Chadwick et al., Chem. Commun., 2010, 46, 4088-4090) testent les zéolites Theta-1, ZSM-23, ferriérite (Si/Al=20) et ZSM-5 (10MR) en déshydratation/isomérisation à 400°C pour réaliser la déshydratation et isomérisat ion simultanée du n-butanol pour l'obtention d'isobutène. Ils mettent en évidence une perte d'activité isomérisante de la ferriérite pour la formation d'isobutène au cours du temps et l'attribuent à un effet négatif de l'eau formée par la réaction de déshydratation. Ceci n'est pas observé avec les autres zéolithes et la ferriérite est la zéolite qui montre la stabilité la plus dégradée.

## OBJET ET INTERET DE L'INVENTION

L'invention est telle que définie dans la revendication 1.

L'invention concerne un procédé de déshydratation isomérisante d'une charge comprenant un monoalcool primaire, seul ou en mélange, de formule R-CH$_2$-OH, dans lequel R est un radical alkyl non linéaire de formule générale C$_n$H$_{2n+1}$ où n est un entier compris entre 3 et 20, ledit procédé opérant en phase gaz à une température moyenne pondérée comprise entre 275°C et 400°C, de préférence 300-400°C, à une pression comprise entre 0, 3 MPa et 1 MPa, de préférence 0,5 MPa et 1 MPa, et à une PPH (poids par poids par heure) comprise entre 5 et 10 h-1, de préférence 7-10 h-1, en présence d'un catalyseur comprenant au moins un liant silicique et au moins une zéolithe présentant au moins une série de canaux dont l'ouverture est définie par un anneau à 8 atomes d'oxygène (8MR), procédé dans lequel de la charge vaporisée entrant dans le réacteur présente une teneur pondérale en eau de 4% à 35%, de préférence de 4 à 15%, et de façon très préférée de 6-15%.

Le procédé selon l'invention permet de produire rapidement un mélange d'alcènes riche en alcènes linéaires. En effet, par le choix de la quantité d'eau présente dans la charge entrant au réacteur et du catalyseur zéolithique, il est obtenu une conversion très élevée (de plus de 98%) de l'alcool. La sélectivité en alcènes linéaires est améliorée par rapport à un procédé sans eau et la sélectivité en alcènes totaux est supérieure à 97%.

Un autre avantage est que la durée de stabilisation du rendement en butènes est quasiment immédiate.

La stabilité thermique de l'alcool est elle aussi largement améliorée en présence d'une proportion optimisée d'eau apportée par la charge. La sélectivité en produits secondaires non désirés (tel que l'isobutyraldéhyde) est limitée notamment à haute température (à 300-400°C, ou encore mieux à 350-400°C).

Ces effets bénéfiques résultent de l'action protectrice de l'eau envers ledit monoalcool. Elle évite probablement la dégradation thermique de l'alcool, et permet probablement de réduire la formation de coke non sélectif, ce qui protège le catalyseur d'une dégradation importante en maintenant sa sélectivité.

Cet ajout permet aussi de limiter la formation de produits secondaires non désirés (telles que des aldéhydes), produits qui pourraient se former en l'absence de catalyseur sur les parois métalliques, par exemple dans les lignes amenant la charge au réacteur.

## DESCRIPTION DÉTAILLÉE DE L'INVENTION

### Charge

Conformément à l'invention, la charge à traiter dans le procédé selon l'invention comprend au moins un alcool de formule R-CH$_2$-OH, R étant un groupe alkyl non linéaire de formule générale C$_n$H$_{2n+1}$ où n est un entier compris entre 3 et 20. Dans un mode de réalisation particulier, n est un entier compris entre 3 et 10.

L'alcool primaire est préférentiellement choisi parmi l'isobutanol ou le 2-methyl-1-butanol, seul ou en mélange. Très préférentiellement, l'alcool primaire est essentiellement de l'isobutanol. De préférence la charge comprend l'isobutanol comme seul alcool.

La charge peut provenir de procédés chimiques ou biochimiques, par exemple fermentaires. En particulier, cette charge peut être issue de procédés de fermentation de biomasse lignocellulosique.

Ladite charge peut également comprendre des impuretés organiques (telles que du méthanol, de l'éthanol, du n-butanol, des aldéhydes, des cétones, et les acides carboxyliques correspondant, par exemple l'acide furanique, acétique, isobutyrique).

A la charge à traiter, il est éventuellement ajouté de l'eau de façon à ce que la charge vaporisée entrant dans le réacteur présente une teneur pondérale en eau de 4-35%, de préférence de 4-15%, et de façon très préférée de 6-15%. De façon très préférée, la teneur pondérale en eau est de 4-10% et 6-10% .

Généralement, l'eau est ajoutée à la charge à traiter en amont du réacteur, la charge à traiter étant à une température inférieure à 300°C, voire i nférieure à 275°C. Elle est ajoutée dans la charge à traiter vaporisée, ou le mélange est vaporisé.

La charge entrant dans le réacteur est sous forme gazeuse. Elle comprend, et de préférence est constituée, dudit monoalcool, des impuretés et de l'eau.

La charge entrant au réacteur comprend au moins 40% et généralement de 55 à 96% poids dudit alcool. Lesdites impuretés représentent au plus 10%pds de la charge entrant au réacteur, voire au plus 5%.

Du fait de cette présence d'eau en quantité ciblée, le pré-cokage du catalyseur, et notamment du catalyseur à base de ferriérite, s'est avéré inutile ; le procédé selon l'invention opère donc sans pré-cokage. Le pré-cokage est une opération visant à sélectiver le catalyseur via un dépôt de coke organique provenant soit d'une exposition du catalyseur à la charge habituelle, souvent dans des conditions plus sévères que les conditions opératoires choisies pour la réaction de déshydratation de l'alcool, soit par le recours à une charge particulière. L'opération était, avant l'invention, un préalable au fonctionnement habituel de l'unité.

**Procédé**

Le procédé (réacteur) est opéré en phase gaz, à une température moyenne pondérée comprise entre 275°C et 400°C, de préférence 300-40 0°C, à une pression comprise entre 0,3 MPa et 1 MPa, de préférence 0,5 MPa et 1 MPa, ou encore 0,3-0,9 MPa ou encore 0,5-0,9MPa, à une PPH comprise entre 5 et 10h-1,de préférence 7-10 h-1.

Par PPH, on entend « Poids par Poids par Heure », c'est-à-dire le débit massique d'alcool primaire dans la charge en entrée de réacteur divisé par la masse de catalyseur dans ledit réacteur. Cette notion est également parfois désignée sous son acronyme anglais de WHSV, ou « Weight Hourly Space Velocity ».

Par température moyenne pondérée (noté TMP), on entend la moyenne de la température dans le lit catalytique, le lit étant l'ensemble des lits présents dans le réacteur, lits dans lesquels se déroule la réaction catalytique, calculée le long de l'axe de l'écoulement dans ledit lit. Soit un lit de longueur L et de surface S, le mélange réactif s'écoulant le long de l'axe longitudinal x de ce lit, l'entrée dans le lit catalytique formant l'origine de l'axe (x=0), la température moyenne pondérée, noté TMP, s'exprime selon la formule suivante :

$$\text{TMP} = \frac{1}{L}\int_0^L T(x)dx$$

La réaction étant endothermique et le réacteur opérant soit en mode isotherme, soit en mode adiabatique, la température moyenne pondérée sera représentative de la température de réaction.

La réaction se déroule dans un ou plusieurs réacteurs et chaque réacteur est opéré dans des conditions identiques. La TMP de chacun des réacteurs est ajustée à une valeur comprise entre 275°C et 400°C. Ainsi, dans la suite de l'exposé, le terme « le réacteur » désigne aussi bien le réacteur de cette étape lorsque celle-ci ne comprend qu'un réacteur, que chacun des réacteurs de cette étape, lorsque celle-ci comprend plus d'un réacteur. Ledit catalyseur est disposé dans un ou plusieurs lits fixes, lesquels peuvent être opérés en écoulement ascendant, descendant ou radial.

La réaction de déshydratation étant endothermique, l'apport en calories est réalisé par tout moyen de chauffage connu de l'homme du métier.

Avant mise en contact avec la charge à traiter, le catalyseur est activé par tout moyen connu de l'homme du métier, par exemple par traitement thermique sous air.

**Catalyseur**

Conformément à l'invention, le catalyseur mis en œuvre comprend une zéolithe présentant au moins une série de canaux dont l'ouverture est définie par un anneau à 8 atomes d'oxygène (8MR) telle que définie dans la classification "Atlas of Zeolite Structure Types, Ch. Baerlocher, L. B. Mc Cusker, D.H. Olson, 6ème Edition, Elsevier, 2007, Elsevier, p142". Cette zéolithe est mise en forme dans un liant.

Selon un mode de réalisation particulier, la zéolithe peut également avantageusement contenir au moins une série de canaux dont l'ouverture de pores est définie par un anneau contenant 10 atomes d'oxygène (10 MR), tel que FER ou MFS.

Ladite zéolithe est avantageusement choisie parmi les zéolithes ayant des canaux 8 et 10MR telles que les zéolithes de type structural FER et MFS, prises seules ou en mélange. La zéolite est plus avantageusement choisie dans le type FER parmi les zéolithes ferrierite, FU-9, ISI-6, NU-23, ZSM-35 et pour le type MFS, c'est la zéolite ZSM-57, prises seules ou en mélange. Ladite zéolithe est très avantageusement de type FER et de préférence c'est la ferrierite. De préférence, ladite zéolite est constituée de ferrierite. De préférence, elle n'a pas subi de traitement pour introduire des éléments alcalins, alcalino-terreux ou autres. Elle peut néanmoins avoir été désaluminée. Elle est sous forme H (hydrogène) ou $NH_4$ (ammonium).

De façon préférée, la ferrierite a un rapport molaire Si/Al de 8 à 70, de préférence choisi entre 10 et 50.

La teneur en zéolite dans le catalyseur est comprise entre 50 et 90% poids, de préférence entre 60 et 80% poids.

Le catalyseur comprend également un liant silicique, généralement le liant est à base de silice, en particulier de silice amorphe.

De préférence, le liant silicique est constitué de silice (aux impuretés près, celles-ci n'ayant pas d'effet catalytique).

La teneur en liant dans le catalyseur est comprise entre 10 et 50% pds, de préférence entre 20 et 40%.

De manière très avantageuse, le catalyseur est constitué d'au moins une zéolite ayant au moins une série de canaux dont l'ouverture est à 8 atomes d'oxygène (8MR) et un liant silicique. De préférence, ledit catalyseur est constitué de zéolithe ferrierite et de liant silicique. De préférence, ledit catalyseur est constitué de zéolithe ferriérite et de silice, et en particulier de silice amorphe. Le catalyseur peut éventuellement contenir des impuretés en faible quantité n'ayant pas d'effet technique sur la conversion /sélectivité du catalyseur.

Le catalyseur est mis en forme, de préférence sous forme d'extrudés cylindriques, multilobés, de billes ou tout autre méthode connue de l'homme du métier à l'exception de poudre.

En effet, le liant est nécessaire pour obtenir une porosité hiérarchisée qui permet d'alimenter en réactif la plus grande partie des cristaux de zéolithes sans subir une perte de charge importante par mètre de lit catalytique. De plus, le liant a pour fonction d'espacer les sites actifs de façon à diminuer l'occurrence de réaction biomoléculaires, réaction donnant dans notre cas des produits non désirés.

En outre, le liant est également utile pour donner une résistance mécanique au catalyseur et rendre possible tout au long de son cycle catalytique les opérations de chargement / déchargement et transport des catalyseurs sans perte de matière sous forme de fines.

Procédé de préparation du catalyseur

Ledit catalyseur utilisé dans le procédé selon l'invention est avantageusement préparé selon un procédé de préparation comprenant au moins les étapes suivantes :

1) une étape de mélange d'au moins une poudre de zéolithe, de préférence sous forme protonique ou ammonium, avec au moins un liant silicique, par exemple une poudre de silice amorphe

2) une étape d'ajout d'un solvant, avantageusement de l'eau, et éventuellement d'agent peptisant ; de préférence un agent peptisant est utilisé

3) une étape de mise en forme du mélange pâteux obtenu à l'issue de l'étape 2), par exemple par extrusion

4) une étape de traitement thermique du matériau mis en forme obtenu à l'issue de l'étape 3) à 50-800°C sous air.

Le liant silicique utilisé dans l'étape 1 est bien connu de l'Homme du métier, il est choisi pour son caractère inerte vis-à-vis des conditions opératoires et notamment vis-à-vis de la présence d'eau dans le procédé.

Une source de liant silicique peut être une silice de précipitation ou une silice issue de sous-produits comme les cendres volantes, par exemple les particules silico-alumineuses ou silico-calciques, et les fumées de silice. On pourra avantageusement utiliser une silice colloïdale, se présentant par exemple sous la forme d'une suspension stabilisée, telles que par exemple des produits commerciaux tels que le Ludox® ou les Klebosol®.

Une poudre de silice amorphe peut avantageusement être utilisée dans l'étape 1).

La poudre de zéolite et le liant silicique (de préférence sous forme de poudre) sont avantageusement malaxés en présence d'un solvant (étape 2), de préférence de l'eau dans lequel un agent peptisant peut avantageusement être dissout afin d'obtenir une meilleure dispersion du liant. La consistance de la pâte est ajustée par le biais de la quantité de solvant.

L'agent peptisant utilisé lors de cette étape peut avantageusement être un acide, une base organique ou inorganique tel que l'acide acétique, l'acide chlorhydrique, l'acide sulfurique, l'acide formique, l'acide citrique et l'acide nitrique, seul ou en mélange, l'ammoniaque, une amine, un composé à ammonium quaternaire, choisi parmi les alkyl-éthanol amines ou les alkyl- amines éthoxylées, l'hydroxyde de tétraéthylammonium et le tétraméthylammonium.

L'agent peptisant peut avantageusement être choisi parmi les bases minérales telles que la soude ou la potasse.

Lors de l'étape 3 de mise en forme, la pâte malaxée est extrudée au travers d'une filière dont la géométrie va imposer la forme du catalyseur.

## **EXEMPLES**

L'étape de déshydratation est réalisé sur une unité de test catalytique à 1 (ex 1) ou 2 réacteurs (ex 2-4) comprenant chacun un lit fixe fonctionnant en mode « down flow », c'est-à-dire en écoulement descendant. Le catalyseur, est chargé sous forme d'extrudés de longueur 2 à 4mm dans chaque réacteur inox 316L de diamètre interne de 13mm. Le catalyseur est ensuite activé à 450°C sous 6l/h d'air pendant un palier d'une heure, après une montée en température de 10°C/min, la température est ensuite abaissée à la température de test sous 6l/h d'azote afin d'éliminer l'air présent dans le système avant injection de la charge alcool.

A la charge d'isobutanol sec, de l'eau est ajoutée. La charge est un mélange isobutanol/eau en rapport massique variable. Elle est vaporisée dans les lignes chauffées à 150-180°C en amont du premier réacteur puis injectée dans le réacteur catalytique. La pression est maintenue à 8 bars.

L'analyse de l'effluent total est effectuée en sortie de réacteur sur un chromatographe en phase gazeuse en ligne équipée de deux colonnes, ce qui permet de déterminer la conversion de l'isobutanol, les sélectivités en différents produits et notamment la sélectivité en butènes et la fraction de butènes linéaires dans la coupe butènes, fraction que l'on cherche à maximiser. L'analyseur permet aussi de mesurer la sélectivité en produits secondaires tels les produits contenant 5 atomes de carbone ou plus (dénommés C5+), les alcanes, les acides carboxyliques ou les éthers. La mesure de la conversion moyenne atteinte pendant les 24h du point retour après 72h de test est comparée à la conversion moyenne pendant les 24 premières heures à pph $7h^{-1}$ et permet d'évaluer la perte d'activité au cours du test.

La PPH correspond au poids horaire de charge injectée relativement au poids du catalyseur.

Les figures sont relatives aux exemples.

Exemple 1 ( 0% non-conforme, 7% conforme)

Le catalyseur A est préparé par mélange de 70% de poudre de ferrierite commerciale sous forme ammonium présentant un ratio atomique Si/Al de 20 et de 30% d'une source de silice commerciale, et 9% d'une source de silice commerciale (les fractions massiques sont calculées par rapport à la masse sèche totale des poudres) par malaxage avec une solution aqueuse de triethylammonium TEAOH, extrusion, séchage puis calcination.

Le catalyseur A est mis en œuvre dans le test catalytique tel que décrit ci-dessus, le débit de charge est de 10.5g/h, ce qui correspond à une pph de 7h-1 pour une masse de1.5g de catalyseur chargé.

| Eau dans la charge (% pds) | T (°C) | Conversion (%) | n-butènes/butènes totaux initial(%) | n-butènes/butènes totaux après 72h (%) | Sélectivité initiale en C4= (%) | Sélectivité en C4= après 72h(%) | Sélectivité initiale en isobutyraldéhyde (%) | Sélectivité en isobutyraldéhyde après 72h (%) |
|---|---|---|---|---|---|---|---|---|
| **Non conforme** | | | | | | | | |
| 0 | 300 | 99.1 | 82.4 | 84.1 | 89.9 | 97.8 | 0.06 | 0.06 |
| 0 | 400 | 99.8 | 65 | 77.0 | 88.9 | 98.7 | 0.11 | 0.41 |
| **Conforme** | | | | | | | | |
| 7 | 300 | 98.5 | 82.6 | 84.1 | 94.4 | 97.8 | 0.06 | 0.06 |
| 7 | 400 | 98.9 | 69.8 | 77.0 | 94.3 | 98.8 | 0.06 | 0.07 |

La durée de stabilisation de la sélectivité en produits désirés (butènes totaux et butènes liénaires) est beaucoup plus rapide en présence de 7% pds d'eau dans la charge qu'en présence d'une charge non diluée. En effet la sélectivité initiale en butènes est supérieure à 94% en présence d'eau alors qu'elle inférieure à 90% en l'absence d'eau dans la charge. Des niveaux équivalents de sélectivité en butènes sont atteints seulement après 72h sous charge.

Ceci souligne l'effet de l'eau ajoutée dans la charge sur la sélectivation du catalyseur. La sélectivité en butènes linéaires qui est le produit visé est ainsi largement supérieure en présence d'eau dans la charge.

Le procédé atteint des rendements en butènes et notamment en butènes linéaires bien supérieurs dès l'injection de la charge et ainsi le rendement du procédé est fortement amélioré.

Par ailleurs la sélectivité en produits secondaires non désirés (isobutyraldéhyde) est limitée à haute température en présence d'eau dans la charge.

Exemples 2-3 : tests en conditions isothermes

Exemple 2 à 30 % d'eau (non-conforme)

Le catalyseur A est mis en œuvre dans un test catalytique sous des conditions isothermes. Pour le test, 200 ml de catalyseur sont chargés sous forme d'extrudés dans deux réacteurs (100 ml de catalyseur par réacteur). Le débit de charge est de 770 g/h, ce qui correspond à une pph de 7.0h-1.

La charge est préchauffée avant l'entrée dans le premier réacteur Rx1 pour avoir une température moyenne pondérée du lit catalytique de 350°C. Entre les deux réacteurs la charge est également préchauffée pour assurer une température moyenne pondérée du lit de 350°C au second réacteur.

Le test est conduit avec une charge contenant 30 pds% H2O.

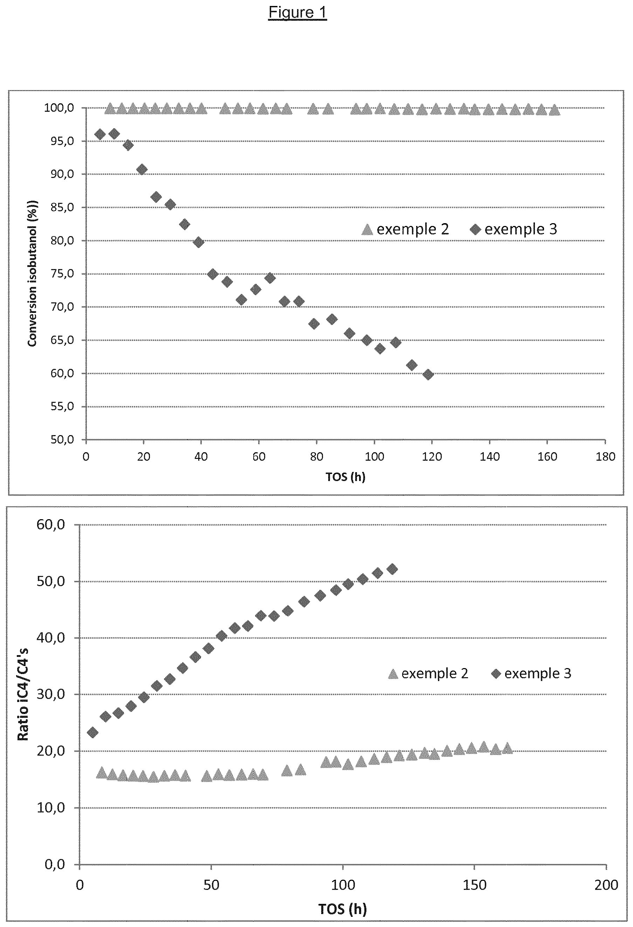

On peut constater (fig.1) une conversion totale de l'isobutanol et une performance stable. Le rapport entre isobutènes sur la totalité des butènes est aussi stable (+4% en 160h).

Exemple 3

Le test est conduit avec une charge 100%pds isobutanol . On peut constater la désactivation très rapide et une augmentation du rapport entre isobutènes sur la totalité des butènes de 30% en 120h.

Exemple 4 : test en conditions isothermes à 10% d'eau (conforme)

Le catalyseur A frais est mis en œuvre dans le test catalytique sous des conditions isothermes. Pour le test, 200 ml de catalyseur sous forme extrudés est chargé dans deux réacteurs (100 ml de catalyseur par réacteur). Le débit de charge est de 770 g/h, ce qui correspond à une pph de 7.0h-1. La charge est préchauffée avant l'entrée dans le réacteur Rx1 pour avoir une température moyenne pondérée du lit catalytique de 315°C. Entre les deux réacteurs la charge est également préchauffée pour assurer la température moyenne pondérée du lit catalytique de 315°C au second réac teur.

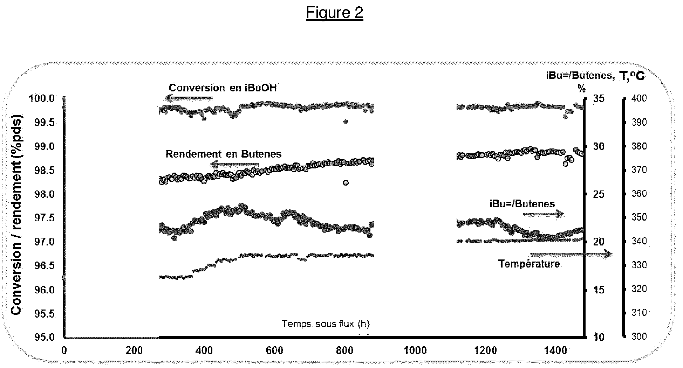

Le test est effectué avec une charge contenant 10 pds% d'eau. Le catalyseur a démontré une performance stable pendant plus que 2000 heures (Fig 2).

**Revendications**

1. Procédé de déshydratation isomérisante d'une charge comprenant un monoalcool primaire, seul ou en mélange, de formule R-CH$_2$-OH, dans lequel R est un radical alkyl non linéaire de formule générale C$_n$H$_{2n+1}$ où n est un entier compris entre 3 et 20, ledit procédé opérant

   - en phase gaz à une température moyenne pondérée comprise entre 300°C et 400°C, à une pression comprise entre 0, 5 MPa et 0,9 MPa et à une PPH (poids par poids par heure) de 7 h-1,
   - en présence d'un catalyseur comprenant au moins un liant silicique et au moins une zéolithe présentant au moins une série de canaux dont l'ouverture est définie par un anneau à 8 atomes d'oxygène (8MR), la zéolithe étant de type FER, le catalyseur n'étant pas pré-coké,
   - procédé dans lequel de la charge vaporisée entrant dans le réacteur présente une teneur pondérale en eau de 6% à 15%.

2. Procédé selon la revendication précédente dans lequel ladite zéolithe est la ferrierite.

3. Procédé selon l'une des revendications précédentes dans lequel la ferrierite a un rapport molaire Si/Al de 8 à 70, de préférence choisi entre 10 et 50.

4. Procédé selon l'une des revendications précédentes dans lequel la teneur en zéolite dans le catalyseur est comprise entre 50 et 90% poids, de préférence entre 60 et 80% poids.

5. Procédé selon l'une des revendications précédentes dans lequel le catalyseur contient, et de préférence est constitué par une ferriérite et un liant silicique.

6. Procédé selon l'une des revendications précédentes dans lequel le catalyseur est constitué de zéolithe ferriérite et de silice amorphe.

7. Procédé selon l'une des revendications précédentes dans lequel l'alcool primaire est l'isobutanol ou le 2-methyl-1-butanol, seul ou en mélange, et de préférence l'alcool est l'isobutanol.

**Patentansprüche**

1. Verfahren zur isomerisierenden Dehydrierung eines Einsatzmaterials das einen primären Monoalkohol, allein oder im Gemisch, mit der Formel $R\text{-}CH_2\text{-}OH$ umfasst, wobei R ein nicht geradkettiger Alkylrest mit der allgemeinen Formel $C_nH_{2n+1}$ ist, in der n eine ganze Zahl zwischen 3 und 20 ist, wobei das Verfahren durchgeführt wird

- in Gasphase bei einer gewichteten mittleren Temperatur zwischen 300 °C und 400 °C, bei einem Druck zwischen 0, 5 MPa und 0,9 MPa und bei einer WHSV (Raumgeschwindigkeit) von 7 h-1,
- in Gegenwart eines Katalysators, der mindestens ein siliciumhaltiges Bindemittel und mindestens einen Zeolithen umfasst, der mindestens eine Reihe von Kanälen aufweist, deren Öffnung durch einen Ring mit 8 Sauerstoffatomen (8MR) definiert wird, wobei der Zeolith vom Typ FER ist, wobei der Katalysator nicht vorverkokt ist,
- wobei bei dem Verfahren in den Reaktor eintretendes verdampftes Einsatzmaterial einen Massegehalt an Wasser von 6 % bis 15 % aufweist.

2. Verfahren nach dem vorhergehenden Anspruch, bei dem der Zeolith das Ferrierit ist.

3. Verfahren nach einem der vorhergehenden Ansprüche, bei dem das Ferrierit ein Molverhältnis Si/Al von 8 bis 70 hat, das bevorzugt zwischen 10 und 50 gewählt ist.

4. Verfahren nach einem der vorhergehenden Ansprüche, bei dem der Zeolithgehalt in dem Katalysator zwischen 50 und 90 Gew.-%, bevorzugt zwischen 60 und 80 Gew.-% beträgt.

5. Verfahren nach einem der vorhergehenden Ansprüche, bei dem der Katalysator ein Ferrierit und ein siliciumhaltiges Bindemittel enthält und bevorzugt daraus besteht.

6. Verfahren nach einem der vorhergehenden Ansprüche, bei dem der Katalysator aus Ferrierit Zeolith und amorphem Siliciumoxid besteht.

7. Verfahren nach einem der vorhergehenden Ansprüche, bei dem der primäre Alkohol Isobutanol oder 2-Methyl-1-butanol, allein oder im Gemisch, ist und der Alkohol bevorzugt Isobutanol ist.

**Claims**

1. Process for the isomerizing dehydration of a feedstock comprising a primary monoalcohol, alone or as a mixture, of formula $R\text{-}CH_2\text{-}OH$, wherein R is a nonlinear alkyl radical of general formula $C_nH_{2n+1}$ where n is an integer between 3 and 20, said process operating

- in the gas phase at a weighted average temperature between 300°C and 400°C, at a pressure between 0.5 MPa and 0.9 MPa and at a WWH (weight per weight per hour) of 7 h-1,
- in the presence of a catalyst comprising at least one silicic binder and at least one zeolite having at least one series of channels, the opening of which is defined by a ring of 8 oxygen atoms (8MR), the zeolite being of FER type, the catalyst not being precoked,
- process wherein the vaporized feedstock entering the reactor has a weight content of water of from 6% to 15%.

2. Process according to the preceding claim, wherein said zeolite is ferrierite.

3. Process according to one of the preceding claims, wherein the ferrierite has an Si/Al molar ratio of 8 to 70, preferably selected between 10 and 50.

4. Process according to one of the preceding claims, wherein the content of zeolite in the catalyst is between 50% and 90% by weight, preferably between 60% and 80% by weight.

5. Process according to one of the preceding claims, wherein the catalyst contains, and preferably consists of, a ferrierite and a silicic binder.

6. Process according to one of the preceding claims, wherein the catalyst consists of ferrierite zeolite and amorphous silica.

7. Process according to one of the preceding claims, wherein the primary alcohol is isobutanol or 2-methyl-1-butanol, alone or as a mixture, and preferably the alcohol is isobutanol.

Figure 1

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009074798 A
- WO 2011113834 A
- WO 2016046242 A1

**Littérature non-brevet citée dans la description**

- **CHADWICK** ; **CHADWICK et al.** *Chem. Commun.*, 2010, vol. 46, 4088-4090
- **CH. BAERLOCHER** ; **L. B. MC CUSKER** ; **D.H. OLSON**. Atlas of Zeolite Structure Types. Elsevier, 2007, 142